# EUROPEAN PATENT SPECIFICATION

(11) **EP 0 490 434 B1**
(45) Date of publication and mention of the grant of the patent: **15.03.2000**
(21) Application number: 91203183.8
(22) Date of filing: 05.12.1991
(51) Int. Cl.: C07H 21/00, A61K 31/70

(54) **Labelled, modified oligonucleotides**
Markierte, modifizierte Oligonokleotiden
Oligonucléotides,marqués et modifiés

(30) Priority: 10.12.1990 NL 9002714
(43) Date of publication of application: 17.06.1992
(73) Proprietor: Akzo Nobel N.V., 6824 BM Arnhem (NL)
(72) Inventor: Kuijpers, Wilhelmus Henricus Arnoldus, NL-5623 CK Eindhoven (NL); Kaspersen, Franciscus Michael, NL-5384 BB Heesch (NL); van Boekel, Constant Adriaan Anton, NL-5345 LX Oss (NL)
(74) Representative: Hermans, Franciscus G.M.

(56) References cited:
- WO-A-88/05077
- WO-A-89/05853
- WO-A-89/06702
- WO-A-89/10140
- WO-A-89/12110
- WO-A-90/10448
- WO-A-90/12802
- CHEMICAL REVIEWS. vol. 90, no. 4, June 1990, EASTON US pages 543 - 584; E. UHLMANN ET AL: 'Antisense Oligonucleotides: A New Therapeutic Principle'
- CHEMICAL ABSTRACTS, vol. 112, no. 11, 12 March 1990, Columbus, Ohio, US; abstract no. 95109, R. HIRSCH ET AL: 'DNA-Antibody Conjugates, Their Preparation and Use in Integration of Foreign DNA into Cells' page 421 ;column 2 ;
- J Am Chem Soc 111 (1989) 9117-9
- TIBTECH 6 (1988) 246-251
- CANCER RES 54 (1994) 3479-86

## Description

The invention relates to modified oligo-nucleotides which are radioactively labelled.
Radioactively labelled oligonucleotides are known, for instance as probes used in hybridization assays.
The use of such oligonucleotides in vivo generally will not be possible, because unmodified DNA and RNA are quickly metabolized in vivo. For in vivo purposes modified oligonucleotides have been described in E. Uhlmann and A. Peyman: Chemical Reviews, 90, 543 (1990).

In so-called "anti-sense oligonucleotide" therapy, synthetic, modified DNA or RNA fragments, which may or may not be conjugated with enzymes or cytostatics, are directed towards complementary nucleic acid fragments (DNA or RNA) which are present in the "target" cells, for example of tumours or cells infected by a virus. Modification of the anti-sense oligonucleotide is necessary for the desired stability in circulation (inter alia, they must be able to withstand endogenous enzymes) and, in addition, the penetration in cells and tissues can be improved as a result of the modification. The anti-sense oligonucleotide is complementary to a nucleic acid in the target cell and may inhibit DNA transcription, mRNA translation, or (viral) nucleic acid replication.
Up to now "anti-sense oligonucleotides" have not been conjugated with chemical groups which are labelled with radioactive isotopes which can be used in radio-immunotherapy.

The present invention relates to modified "anti-sense oligonucleotides" which are labelled with isotopes which can be used therapeutically (radioactive anti-sense nucleotide: RAN) and the use of RANs in anti-cancer and anti-viral therapy. Moreover, the RANs can be labelled in such a way that the localisation of the complementary nucleic acid (fragment) is taken into account. If the RAN has to have an intracellular action, isotopes which emit Auger electrons are preferred (such as ⁷⁷Br, ⁸⁰Br, 125_{I} and 123_{I}), while in the case of extracellular localisation alpha- or beta-emitting isotopes (such as 90_{Y}, 131_{I,} ¹⁸⁶Re, ²¹¹At and Bi isotopes) are preferred. The latter category of isotopes is also preferred if the RAN has to reach its "target" both inside and outside the cell.
The RANs may be prepared according to any known technique for synthesis of oligonucleotides, for example as described in E. Uhlmann and A. Peyman: Chemical Reviews, 90, 543 (1990). Natural sugar backbones (ribose and deoxyribose) or their enantiomers may be chosen, possibly suitably modified, for instance 2'-O-methyl ribose, but it will also be possible to use stereoisomers (enantiomers α instead of β nucleosides) to choose other backbones such as deoxyglucose, as long as they can be made more resistant to endogenous enzymes and as long as they can provide for base pairing of the oligonucleotides, with a sufficient Tm (melting temperature).
Also the phosphate binding between the sugars may be replaced by a more stable bond (in vivo), such as -CH₂-SO₂-CH₂-, or -O-CH₂-O-.
The bases may be the natural bases (A,U,C,G,T,), but they may also be rare bases such as xanthine, hypoxanthine and bases such as described in Benner et al., J.Am.Chem.Soc.Vol.111 p21,1989.
The RANs may be either linear oligonucleotides or circular oligonucleotides.

Preferred RANs are radioactively labelled, modified oligonucleotides of type I. 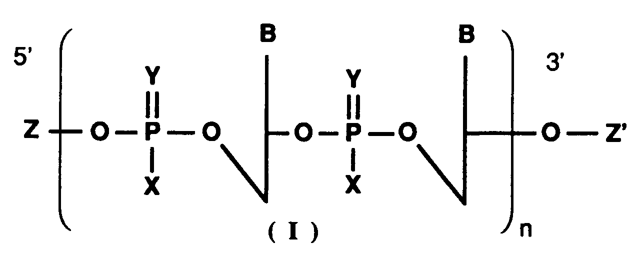 wherein:
- n =: 5 - 20
- B =: nucleoside base (C, G, A, T, U) or analogous bases, optionally labelled with a radioactive halogen.
- X =: O⁻, S⁻, N-dialkyl, O-alkyl, alkyl and/or combinations thereof.
- Y =: O and/or S.
- Z,Z' =: a chemical group which contains a radioactive isotope, and/or Z,Z' is H, alkyl, acyl or nucleoside.

The base sequence (location of B) is determined by the base sequence of the "target nucleic acid fragment", the RAN being complementary to the sequence of the latter (for instance C-G and A-T as complementary bases).

Another aspect of the invention provides a conjugate of a targeting moiety such as an antibody or a fragment or a derivative thereof, directed to an antigen associated with a target cell and a modified oligonucleotide which is complementary to a RAN. It is also possible to choose any other member of a specific binding pair which complementary member is associated with a specific group of target cells as a targeting moiety , such as ligands for receptors and the like. The conjugate can be administered to a subject; it will localize at the target site and after allowing sufficient time for said localization a RAN is administered, which will be bound by the complementary modified oligonucleotide in the conjugate.

The present invention therefore provides a pharmaceutical preparation for the targeted delivery of a therapeutic entity to target cells, characterised in that it comprises (a) a dosage unit comprising a conjugate of a targeting moiety having specificity for the target cell and a modified oligonucleotide; and (b) another dosage unit comprising a labeled modified oligonucleotide that is complementary to the oligonucleotide of the conjugate and that is labeled with the therapeutic entity; and (c) both dosage units further comprising a suitable medium for administration.

The pharmaceutical preparations of the invention will greatly enhance the possibilities of targeted therapy as used in for instance the combat of (auto) immune diseases, viral infections and tumours. Such a pretargeting programme is shown in scheme 1.

### Scheme 1 A and B: Therapeutic applications of radiolabelled antisense nucleotides (RANs).

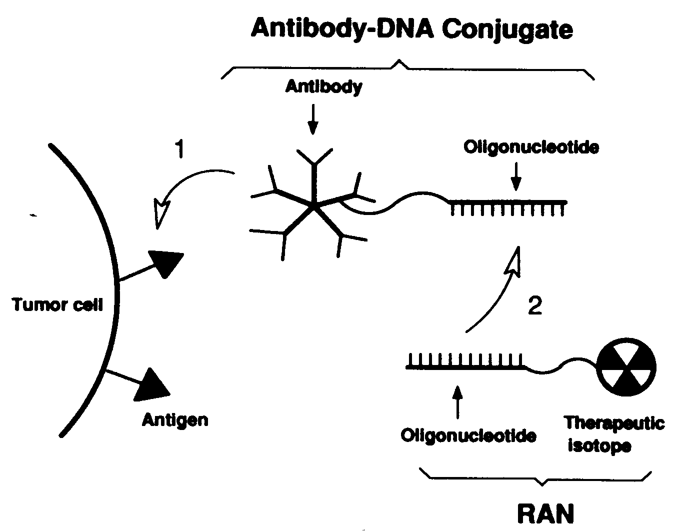
Antibody-DNA Conjugate
Antibody
1
Oligonucleotide
Tumor cell
2
Antigen
Oligonucleotide
Therapeutic
isotope
RAN

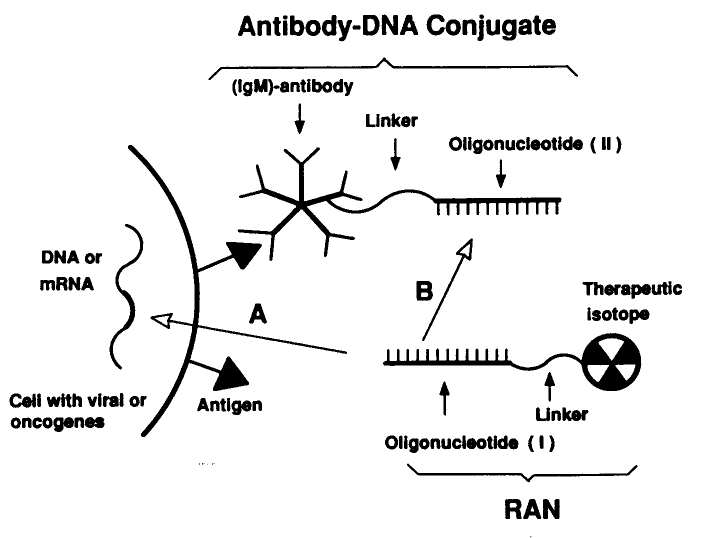
Antibody-DNA Conjugate
(IgM)-antibody
Linker
Oligonucleotide ( II )
DNA or
mRNA
B
Therapeutic
isotope
A
Cell with viral or
oncogenes
Antigen
Linker
Oligonucleotide ( I )
RAN

The use of specific antibodies in tumour therapy is known. However, the use of radioactively labelled antibodies is restricted by the slow uptake in the "target" tissues, as a result of which there is an unfavourable ratio of exposure to radiation between diseased and healthy tissues.
A so-called "pre-targeting programme" is used in order to improve this unfavourable ratio. In this programme non-radioactive bi-functional targeting moieties such as antibody conjugates are dispatched to the diseased tissues (initial binding) and subsequently, at the time when the antibodies are distributed in an optimum ratio between diseased and healthy tissues, a radioactively labelled ligand is administered which is distributed rapidly over the tissues and adheres to the second binding site of the antibody. However, possibilities for pre-targeting which are mentioned in the literature still have their limitations, inter alia as a result of, on the one hand, the low affinity of the second specific binding and, on the other hand, due to the use of substances foreign to the body, such as avidin (see Goodwin et al., J. Nucl. Med. 28, 722 (1987) and 29, 226 (1988)). The use of RANs has the advantage that RANs i) have a strong similarity to substances found in the body, ii) enter into strong, specific bindings, iii) distribute rapidly and well over tissues and iv) the residence time in the human body can be optimised by means of the nature of the chemical modification.
If RANs are directed towards oligonucleotides bound to targeting moieties such as antibodies, the bound oligonucleotide must be complementary to the RAN and of type II: 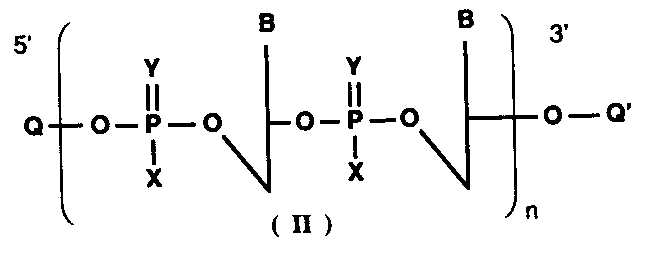 in which:
- n =: 5 - 20.
- B =: nucleoside base.
- X =: O⁻, S⁻, N-dialkyl, O-alkyl, alkyl and/or combinations thereof.
- Y =: O and/or S.
- Q,Q' =: a chemical fragment which is suitable for conjugation with targeting moiety. This fragment contains a reactive aldehyde, NH₂ or SH group, and/or Q,Q' is H, alkyl, acyl or nucleoside.
Such groups (Q,Q') and conjugation techniques which are needed to couple Q or Q' to targeting moiety are described in the review article by J. Goodchild: Bioconjugate Chemistry 1, pages 165 to 187 inclusive (1990).
Optionally the type II nucleotide may be labelled with an isotype for imaging.

It is preferred to couple several (type II) oligonucleotides to the targeting moiety, in order to increase the number of binding sites for the oligonucleotide of type I. The greatest effectiveness is achieved if the oligonucleotide sequence corresponds to a sequence which is unique for an intracellular oncogene or viral gene.
RANs are prepared by labelling chemically synthesized, modified oligonucleotides (type I) with the desired radioactive isotope. The synthesis of modified nucleotides which are suitable both for the RAN (type I) and for type II is effected in accordance with known methods such as described in Chemical Reviews 90 pages 543 to 584 inclusive (1990), modifications such as methylphosphonates, phosphorus amidates, phosphorus thioates and alkylphosphotri-esters being preferred, which - preferably - are incorporated in combination with naturally occurring phosphodiester bands.
Because the last mentioned oligonucleotides are usually prepared with the aid of so-called "solid phase" synthesis, the groups to be labelled are preferably positioned at the 5' end. Examples of these groups in the case of labelling with radioactive halogens are: 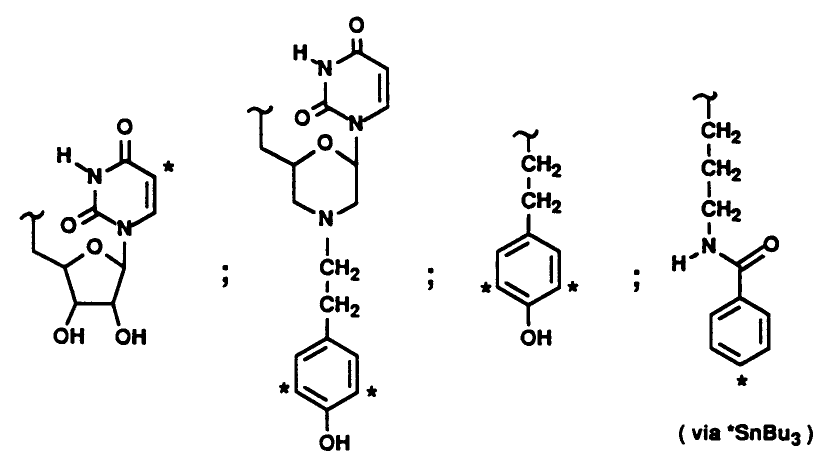 where * is radioactive halogen.
An alternative is direct halogenation of the nucleic acid bases.

If radioactive metals are used, Z/Z' is a complex of a radioactive metal and a chelator such as DTPA macrocyclic chelating agents, NₓSₓ chelating agents and similar substances, such as:

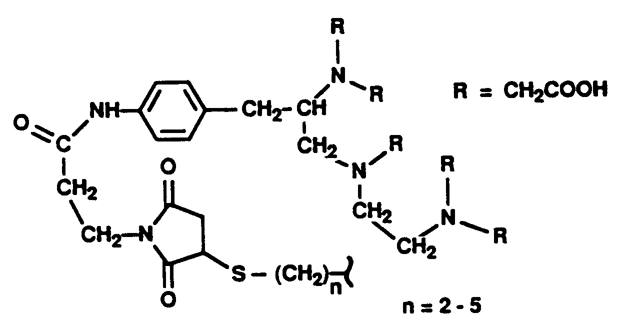

### EXPERIMENTAL

In antisense therapy, various DNA modifications have been introduced, mainly to enhance the stability of the oligonucleotide against nucleases and to improve the cellular uptake. Most commonly used are backbone-modified DNA analogues such as phosphorothioate, methylphosphonate and alkyl phosphotriester (inter alia phosphate-methylated DNA) oligonucleotides. In all these cases, however, introduction of modified phosphate groups should not lead to poorer solubility of the oligonucleotide in water or to a decrease of the stability of the complex with the target sequence.
As mentioned, both DNA fragments, the RAN and the fragment conjugated to the targeting moiety, have to be modified to protect them against nuclease degradation. The main nuclease activity in the blood origins from 3'-exonucleases. Therefore, modification of the phosphodiester(s) at the 3'-end of the oligonucleotide has shown to be important. As the oligonucleotide bound to the targeting moiety should be stable for quite a long period (2-3 days), this fragment should be more severely modified than the RAN, but not to an extent that the material becomes immunogenic. For our purposes, we chose the methyl-phosphonate modification. Methylphosphonate linkages can be easily introduced into DNA fragments using standard (phosphoramidite) DNA chemistry.
Furthermore, this modification is stable towards the chemical conditions necessary for conjugation and radiolabelling and the introduction of neutral internucleoside linkages diminishes the non-specific binding of the highly charged oligonucleotides to positively charged blood proteins.

In principle, partially phosphate-methylated DNA fragments fulfill our goals as well. We were successful in developing a synthetic strategy for the preparation of well-defined partially phosphate-methylated DNA. The lability of the methyl phosphotriester in this modification requires mild conditions during conjugation and radiolabelling. Otherwise demethylation of the phosphotriester or other side-reactions may occur.

### Example 1.

Using an Applied Biosystems DNA synthesizer (model 381A), the following complementary DNA fragments I and II, bearing 2 and 5 methylphosphonate linkages respectively, were synthesized (obtained after deprotection using ammonia in methanol during 3 days and purification with the aid of gel permeation chromatography)
- I :: U5'-5'Gp(Me)CCGGCGCAAGCGp(Me)C 3'
- II :: U5'-5'Gp(Me)CGCTp(Me)TGCGp(Me)CCGp(Me)Gp(Me)C 3'

p(Me) denotes a methylphosphonate which was introduced using suitably protected methylphosphonamidites.

### Duplex stability.

The stability of a duplex between two complementary DNA strands is expressed as the melting temperature Tm. This temperature, at which half of the helical structure is lost, can be monitored by measuring the absorbance at 260 nm as a function of temperature. The Tm-value for the DNA duplex between I and II in 0.1 M NaCl, 0.01 M Tris.HCl (pH = 7.0) was found to be 57°C. Under physiological conditions in vivo (37°C) an efficient hybridization will occur.

At the 5'-end of the modified DNA fragments (I, II) a 5'-5' linked uridine nucleotide was introduced. This uridine moiety offers, after oxidation of the cis diol, the possibility to incorporate various amino-containing linkers via reductive amination (Scheme 2). Obviously the highest modified DNA fragment II was used for conjugation with the antibody. 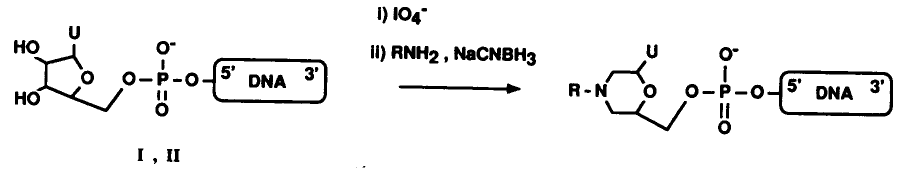

i) IO4⁻

ii) RNH2 , NaCNBH3


I , II
- Scheme 2:: Incorporation of amino-containing linkers via 5'-5' linked uridine.

### Example 2.

Preparation of radiolabelled oligonucleotides (RANs).

A RAN was obtained by radioactive labelling of the synthetic nucleotide (I).

### U 5'-5'-Gp(Me)CCGGCGCAAGCGp(Me)C 3'

By reacting the oligonucleotide with sodium periodate (0.05 M ; 30 eq.) in sodium acetate buffer (0.1 M, pH = 4.75) during 1 hour at 0°C in the dark, the ribose (of U) was converted to a dialdehyde. After removing the excess periodate on a Sephadex G-10 column, the dialdehyde was aminated in a solution of 0.05 M tyramine in 0.1 M phosphate buffer (pH = 6.9)/methanol (2:1 v/v)).
After 30 minutes at room temperature sodium cyanoborohydride (0.1 M; 30 eq.) in methanol was added. The reduction was allowed to proceed overnight at room temperature, after which an additional amount (30 eq.) of sodium cyanoborohydride was added (1 hour, room temperature). The reaction mixture was concentrated and applied to a Sephadex G-25 column using 0.05 M TEAB as eluent. DNA-containing fractions were collected and lyophilized to yield the derivatized oligonucleotide III.

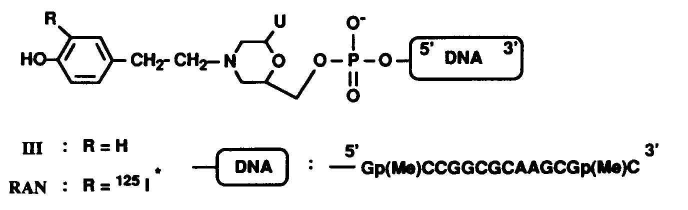
R
HO
CH2-CH2-N
U
O
O-
O-P-O
O
5' DNA 3'

III : R = H
RAN : R = 125 I *
DNA : 5' Gp(Me)CCGGCGCAAGCGp(Me)C 3'

### Scheme 3.

The p-hydroxyphenylethyl moiety of oligonucleotide III could be easily labelled with Na¹²⁵I in the presence of iodogen ^{R}.
Briefly, an Eppendorf reaction tube was coated with iodogen ^{R} (50 µg) by drying an iodogen ^{R} solution in methylenechloride by means of nitrogen. To the reactiontube was added 10 µg Na¹²⁵I solution (≈2 mCi). The iodionation was allowed to proceed for 15 minutes at room temperature with occasional shaking. The RAN was purified on Sephadex PD10 using either PBS buffer or 2 x SSC buffer (for hybridisation studies). The specific activity of the RAN thus obtained was 35 µCi/µg.

### Example 3.

Preparation of antibody-oligonucleotide conjugates.

First, oligonucleotide II was derivatized with an activated thiol linker in an analogous way as described for the derivatization of oligonucleotide. Briefly, oligonuleotide II was oxidized with 0.05 M sodium periodate (12.5 eq.) in 0.12 M sodium acetate buffer (pH = 4.75) during 1 hour at 0°C in the dark. Subsequent to removal of excess periodate (Sephadex G-10) the dialdehyde was aminated with 0.04 M S-pyridylcysteamine. HCl (30 eq.) in 0.1 M phosphate buffer (pH = 8.0)/methanol (2:1 v/v), followed by reduction with sodium cyanoborohydride (5 eq.) in methanol during 16 hours at room termperature. An additional amount of sodium cyanoborohydride (5 eq.) was added before purification of the oligonucleotide on a Sephadex G-25 column using 0.05 M TEAB as eluent. Lyophilization of the DNA containing fractions afforded oligonucleotide IV containing a thiol linker activated as a 2-pyridyldisulfide. According to UV-absorbance of the pyridine-thione released upon reduction of the disulfide, approximately 45% of the oligonucleotide contained the activated thiol linker.

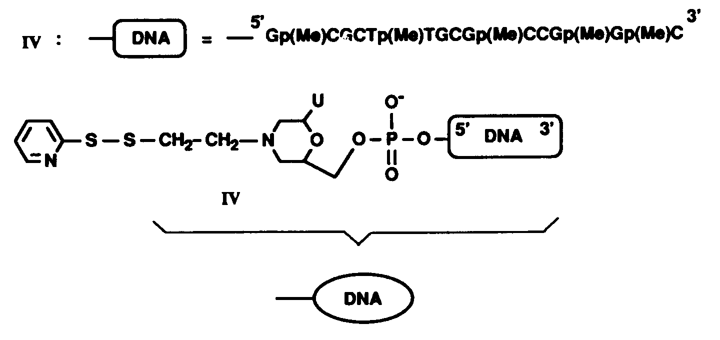
IV : DNA = —5' Gp(Me)CGCTp(Me)TGCGp(Me)CCGp(Me)Gp(Me)C 3'

U
O-
S — S — CH2 — CH2 — N O O — P — O — 5' DNA 3'
N
O
IV


DNA

### Scheme 4.

As disulfide linked protein-DNA conjugates may be insufficiently stable in in vivo systems, we focused on the maleimide coupling strategy. Generally, in this approach the antibody is derivatized with maleimide groups by treatment of the protein with succinimidyl-4-(N-maleimidomethyl)cyclohexane-1-carboxylate (SMCC).

Subsequently, a conjugate with the thiol-containing oligonucleotide is formed via a stable thioether linkage (Scheme 5).

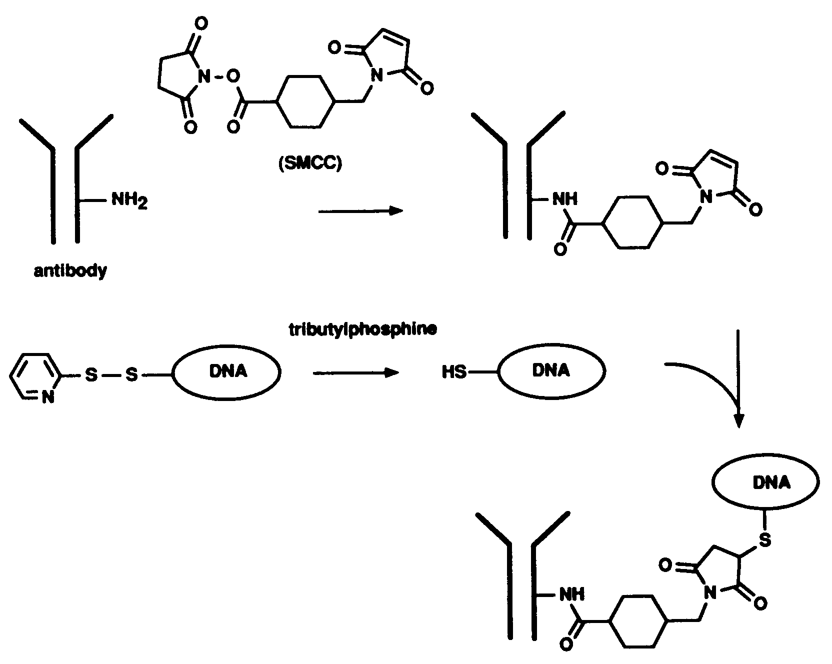

O
O
N-O
N
O
O
O
O
(SMCC)
O
O
N
NH2
NH
O
antibody
O

tributylphosphine
S—S
DNA
HS—
DNA
N

DNA
S
O
N
O
NH
O

### Scheme 5: Preparation of antibody-oligonucleotide conjugates.

### Example 4.

In vitro experiments with the anti-HCG antibody 130A.

Initially, the anti-HCG antibody 130A was used to investigate the proper conditions for conjugation of the oligonucleotide.

### Conjugate preparation. (I)

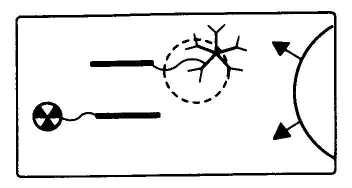

First, the IgG 130A in 0.05 M phosphate buffer (pH = 7.5) was reacted with SMCC (0.01 in DMF) during 1 hour at room temperature in the dark. An average of 10 maleimide groups per antibody was introduced upon treatment with 60 eq. of SMCC. The antibody was purified on a PD-10 column using 0.05 M phosphate/0.1 M NaCl/5 MM EDTA (pH = 6.0) as eluent. After reduction of the 2-pyridyldisulfide activated oligonucleotide in 0.1 M phosphate buffer (pH = 8.0)/methanol (2:1 v/v) was effected by treatment with tributylphosphine (1 eq., 5 min, r.t.), the thiol containing DNA fragment (50 eq. with respect to the antibody) was reacted immediately with the maleimide-derivatized antibody (Scheme 5). The conjugation was allowed to proceed overnight at 4°C, followed by blocking of the unreacted maleimide groups with cysteamine.

The presence of covalently attached oligonucleotides in the conjugate preparation could be demonstrated by IsoElectricFocussing (IEF) and SDS-PAGE. In IEF a shift of the isoelectric point towards lower pH-value was observed, and SDS-PAGE showed an increase in molecular size in comparison with unconjugated IgG 130A.

The antibody-oligonucleotide conjugate could easily be separated from excess of unreacted oligonucleotide by gel filtration on Sephacryl S-100. HR column run at 4°C with PBS as eluent. The fractions containing antibody-conjugate were stored at 4°C.
Based on the 260 nm/ 280 nm absorbance ratio of the purified conjugate a 3:1 molar composition of oligonucleotide and antibody was determined.

### Hybridisation studies. (II)

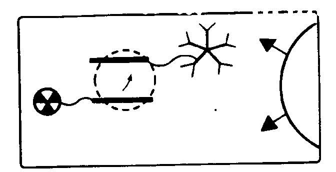

The RAN was allowed to hybridise with the oligonucleotides of the antibody conjugate (10 µg/ml) in 2 x SSC buffer. A slight excess (approx. 1.2 eq.) of RAN was used in comparison with the total amount of conjugated DNA fragments. After 1 hr. hybridisation at room temperature, the mixture was applied on a Sephacryl S-100 column and eluted with PBS buffer at 4°C in order to separate unhybridised RAN from the conjugate/RAN complex. The absorbance at 280 nm and the radioactivity of the eluted fractions were measured (Scheme 6).

### Scheme 6: Gel filtration of IgG130A-DNA/RAN hybridization mixture.

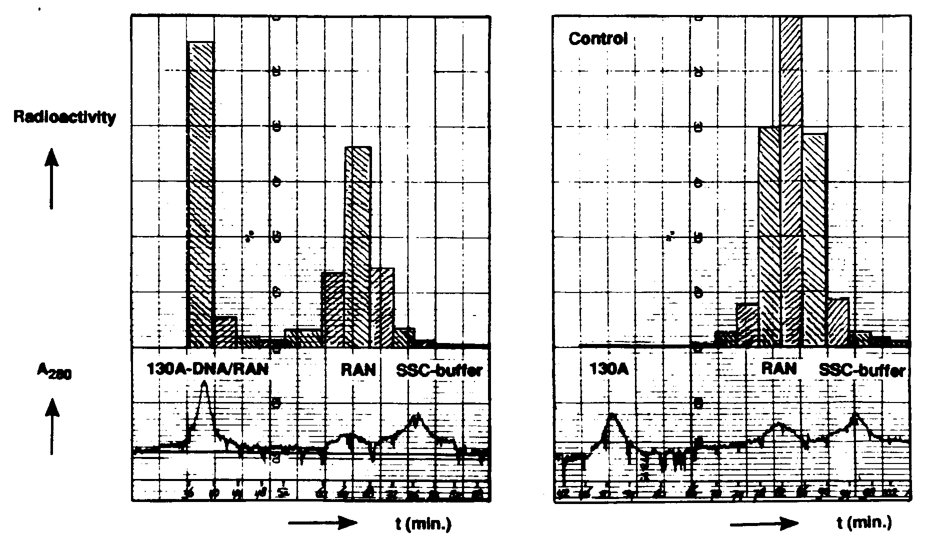

The elution profile showed radioactivity in both the conjugate peak and RAN peak.
Approximately 45% of the eluted radioactivity was present in the peak containing the antibody-conjugate. In order to exclude non-specific binding of the RAN to the anti-body, a control experiment was carried out in which underivatized IgG 130A was incubated with the RAN in the hybridization buffer. Gel filtration of the mixture revealed that less than 0.1% of the registered radioactivity was present in the antibody fraction.

These experiments demonstrate that the RAN is indeed capable to recognize the complementary sequence on the antibody via a specific hybridization mechanism.

### Antigen binding studies. (III)

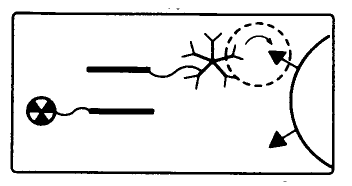

The next question to be answered was whether the antibody-oligonucleotide conjugate had retained its antigen binding properties. To elucidate this issue, part of the hybridization mixture (see above) was incubated with HCG-coated Sepharose beads.
After 90 minutes at room temperature, the mixture was centrifuged and the supernatant was removed. The beads were washed several times with hybridization buffer before the remaining radioactivity on the beads was determined. A 150-fold higher radioactivity was measured relative to the control experiment, in which HCG-Sepharose beads were incubated with a mixture of the RAN and IgG 130A and treated as above. This illustrates that no non-specific binding of the RAN to the antibody (or beads) occurs.

In conclusion, these antigen binding studies clearly indicate that the antibody after conjugation with the oligonucleotide has retained its ability to bind specifically to antigens in vitro.

### Example 5.

In vitro experiments with the human anti-tumour antibody 1688.

Encouraged by the promising results with the IgG 130A, we focused our efforts on the IgM 1688, which, at the moment, is evaluated in radioimmunotherapy of various tumours.

IgM 1688 was activated with maleimide groups by treatment with SMCC as described for the 130A antibody. Since an IgM offers more reactive sites than an IgG, a slighter excess of SMCC was used in comparison with IgG 130A derivatization.
Reaction with 17 eq. of SMCC afforded an average derivatization with 8.5 maleimide groups, as was determined spectrophotometrically. The activated antibody was reacted immediately with freshly reduced (tributylphosphine) oligonucleotide IV (40 eq. with respect to the antibody). After conjugation overnight (4°C) and subsequent blocking of unreacted maleimide groups with cysteamine, the antibody-oligonucleotide conjugate was separated from excess oligonucleotide and other reagents on a Sephacryl S-100 HR column with PBS buffer as eluent at 4°C. Although a shift in the 260 nm/280 nm absorbance ratio was observed for the purified conjugate (0.63) in comparison with IgM 1688 (0.57), a precise quantification of the number of conjugated oligonucleotides was not possible, due to the dominating UV-absorbance of the protein. Based on our experience with the 130A antibody, however, we estimate that 2-3 oligonucleotides are linked to the antibody.

In analogy with the IgG 130A experiments, the IgM 1688-DNA conjugate was incubated with the RAN (approx. 2 eq. with respect to the conjugate) in 2 x SSC buffer to allow hybridisation of the complementary DNA fragments. After a hybridisation period of 1 hr. at room temperature, a S-100 column (PBS buffer, 4°C) was used to separate the antibody containing fraction from unhybridized RAN. Determination of radioactivity in both fractions revealed that 16% of the eluted counts was present in the antibody-conjugate fraction. As with IgG 130A, the control experiment, in which RAN and IgM 1688 were incubated and subsequently separated, showed that negligible non-specific binding of RAN to IgM 1688 had occurred: 0.2% of eluted radioactivity was present in the antibody peak.

These findings were confirmed by a series of dot-blot hybridisation experiments. In this assay a dilution series of the IgM 1688-DNA conjugate was immobilised on a nitrocellulose filter. After blocking the filter with BLOTTO to suppress non-specific binding, it was incubated with RAN during 2 hr. at room temperature in 2 x SSC buffer. Then, the filter was washed and imaged with autoradiography, showing a good dilution pattern.

In a parallel experiment, a similar dilution series of IgM 1688-DNA conjugate was treated with an equal amount of RAN diluted 100-fold with unlabelled oligonucleotide III. The radioactivity spotted in this series perfectly matched the pattern for 100-fold dilution in the first experiment. In the control experiment, in which a nitrocellulose filter immobilised with IgM 1688 was incubated with RAN, only background radioactivity was detected.

Summarizing, these results prove that despite of the huge dimensions of the IgM antibody the RAN is still capable to interact specifically with the conjugated oligonucleotides.

### Antigen binding studies.

The antigen binding capacity of the IgM 1688-DNA conjugate was, as with the IgG 130A conjugate, examined by incubation of Sepharose beads coated with CTA-1, the cognate antigen of IgM 1688, with a hybridisation mixture of conjugate and RAN. In comparison with the control IgM 1688/RAN mixture 17.5-fold radioactivity on the CTA-coated beads was measured.

Apart from the above experiment, in which the RAN was hybridised with the conjugate before the latter had bound to the antigen, an additional test was carried out mimicking the pre-targeting "binding sequence". Thus, first CTA-coated beads were incubated with IgM 1688-DNA conjugate overnight at 4°C (step 1). After removal of excess conjugate and several washings, the beads were incubated with RAN during 2½ hr. at room temperature in 2 x SSC buffer (step 2). Likewise, a competition experiment was performed including hybridisation with the same amount of RAN, diluted 100-fold with unlabelled oligonucleotide III. Subsequent to removal of the supernatant and several washings, the remaining radioactivity on the beads was determined. The results of these experiments are summarized in Table 1.

**Table 1**

| step 1 | step 2 | relative radioactivity |
|---|---|---|
| IgM 1688-DNA | RAN | 10.6 |
| IgM 1688 (control) | RAN | 1.8 |
| IgM 1688-DNA | RAN / DNA(III) (1:100) | 1.4 |
| IgM 1688 (control) | RAN / DNA(III) (1:100) | 1 |

As expected, the highest radioactivity is found for the pre-targeting experiment with undiluted RAN. Moreover, the data clearly demonstrate the competitive behavior of the unlabelled oligonucleotides.

Above experiments (with CTA-coated beads acting as a model for antigen expressing tumor cells) illustrate that a radiolabelled oligonucleotide is able to recognize its complementary sequence on an antibody-conjugate, even when the latter is bound to its antigen. In this manner, pre-targeting based on DNA hybridization has been shown to be successful in vitro.
Human antitumour antibody 16.88 and its antigen are disclosed in EP(A) 0328578.

## Claims

1. A pharmaceutical preparation for the targeted delivery of a therapeutic entity to target cells, characterised in that it comprises
(a) a dosage unit comprising a conjugate of a targeting moiety having specificity for the target cell and a modified oligonucleotide; and
(b) another dosage unit comprising a labelled modified oligonucleotide that is complementary to the oligonucleotide of the conjugate and that is labelled with the therapeutic entity; and
(c) both dosage units further comprising a suitable medium for administration.

2. The pharmaceutical preparation according to claim 1, wherein the therapeutic entity is a therapeutic isotope.

3. The pharmaceutical preparation of claim 1 or 2, wherein the targeting moiety is an antibody or a fragment or a derivative thereof.

4. A use of the pharmaceutical preparation of any one of claims 1-3 for the preparation of a medicament for the treatment of (auto)immune diseases, viral infections and tumours.

## Patentansprüche

1. Pharmazeutisches Präparat für die gezielte Abgabe einer therapeutischen Einheit an Zielzellen, dadurch gekennzeichnet, das es umfasst:
(a) eine Dosierungseinheit, welche ein Konjugat aus einer Zielkomponente mit Spezifität für Zielzellen und ein modifiziertes Oligonukleotid umfasst; und
(b) eine weitere Dosierungseinheit, die ein markiertes modifiziertes Oligonukleotid umfasst, das zu dem Oligonukleotid des Konjugats komplementär ist und das mit der therapeutischen Einheit markiert ist; und
(c) beide Dosierungseinheiten, welche weiterhin ein für eine Verabreichung geeignetes Medium umfassen.

2. Pharmazeutisches Präparat gemäss Anspruch 1, worin die therapeutische Einheit ein therapeutisches Isotop ist.

3. Pharmazeutisches Präparat gemäss Anspruch 1 oder 2, worin die Zielkomponente ein Antikörper oder ein Fragment oder ein Derivat davon ist.

4. Verwendung des pharmazeutischen Präparats nach einem der Ansprüche 1-3 zur Herstellung eines Medikaments zur Behandlung von (Auto-)Immunkrankheiten, viralen Infektionen und Tumoren.

## Revendications

1. Une préparation pharmaceutique pour la libération dirigée d'une entité thérapeutique à des cellules cibles, caractérisée en ce qu'elle comprend :
(a) une unité galénique comprenant un conjugué d'un reste directeur ayant une spécificité pour la cellule cible et un oligonucléotide modifié ;
(b) une autre unité galénique comprenant un oligonucléotide modifié par marquage qui est complémentaire de l'oligonucléotide du conjugué et qui est marqué à l'aide de l'entité thérapeutique ; et
(c) les deux unités galéniques comprenant en outre un milieu convenable d'administration.

2. La préparation pharmaceutique selon la revendication 1, dans laquelle l'entité thérapeutique est un isotope thérapeutique.

3. La préparation pharmaceutique selon l'une quelconque des revendications 1 ou 2, dans laquelle le résidu orientant est un anticorps ou un fragment ou un dérivé de celui-ci.

4. L'utilisation d'une préparation pharmaceutique selon l'une quelconque des revendications 1 à 3, pour la préparation d'un médicament pour le traitement de maladies (auto)immunes, d'infections virales et de tumeurs.
